## Europäisches Patentamt
## European Patent Office
## Office européen des brevets

(19)

(11) Veröffentlichungsnummer: **0 146 491**
**B1**

(12) **EUROPÄISCHE PATENTSCHRIFT**

(45) Veröffentlichungstag der Patentschrift: **16.05.90**

(51) Int. Cl.⁵: **A 61 F 2/32,** G 06 F 15/20

(21) Anmeldenummer: **84730118.1**

(22) Anmeldetag: **05.11.84**

(54) **Verfahren und Vorrichtung zur Herstellung einer Endoprothese.**

(30) Priorität: **03.11.83 DE 3340024**

(43) Veröffentlichungstag der Anmeldung:
**26.06.85 Patentblatt 85/26**

(45) Bekanntmachung des Hinweises auf die
Patenterteilung:
**16.05.90 Patentblatt 90/20**

(84) Benannte Vertragsstaaten:
**AT BE CH DE FR GB IT LI**

(56) Entgegenhaltungen:
**EP-A-0 091 001**
**DE-A-3 215 990**

**BIOMEDIZINISCHE TECHNIK, Band 26, Heft 1,2,
1981, Berlin; C.H.SIEMSEN: "Systematik der
Entwicklung einer Endoprothese von der
Planung bis zur Implantation usw."**

(73) Patentinhaber: **MECRON MEDIZINISCHE
PRODUKTE GMBH
Nunsdorfer Ring 25-27
D-1000 Berlin 48 (DE)**

(72) Erfinder: **Anapliotis, Emmanuel
Tollensestrasse 16
D-1000 Berlin 37 (DE)**
Erfinder: **Kranz, Curt
Landshuter Strasse 5
D-1000 Berlin 30 (DE)**

(74) Vertreter: **Christiansen, Henning, Dipl.-Ing.
Patentanwalt CHRISTIANSEN Pacelliallee 43/45
D-1000 Berlin 33 (DE)**

## Beschreibung

Die Erfindung betrifft verschiedene Verfahren zur Herstellung einer Prothese, wie sie in den Oberbegriffen der Ansprüche 1 bis 4 angegeben sind sowie Vorrichtungen zur Durchführung dieser Verfahren.

Bei der Herstellung von Prothesen und insbesondere von gekrümmten Prothesenschäften stellt deren Anpassung an die individuellen Abmessungen der Hohlräume in den den jeweiligen schaft aufnehmenden Knochen ein Problem dar, das derzeit durch das Vorrätighalten von Schäften in den verschiedensten Abmessungen gelöst wird.

Nachteilig war dabei, daß selbst bei umfangreicher Lagerhaltung nicht allen sich bei der Operation stellenden Anforderungen entsprochen werden konnte und zur individuellen Anpassung vielfach Knochensubstanz zusätzlich beseitigt werden mußte, welche bei exakter Anpassung hätte erhalten bleiben können. Berücksichtigt werden muß dabei auch die Einsetzbarkeit von gekrümmten Prothesenschäften, wobei die zusätzliche Problematik besteht, daß einerseits genügend Raum vorhanden sein muß, um den gekrümmten Prothesenschaft trotz der in der Einführungsöffnung des Knochens ebenfalls vorhandenen Krümmung korrekt einzuführen und andererseits im eingesetzten Zustand einen festen Sitz der Prothese, insbesondere bei zementfreier Implantation sicherzustellen.

Aus der DE 32 13 434 Cl ist auch ein Verfahren bekannt geworden, welches die Herstellung individuell gestalteter Prothesen oder Implantate mittels Computer-Tomographie und Computereinsatz bei der nachfolgenden Berechnung beschreibt. Dabei besteht jedoch ein Problem darin, daß bei gekrümmten Prothesenteilen zwar eine optimal passende Prothese hergestellt, diese aber durch eine vorhandene Knochenöffnung oder einen bestehenden Zugangsweg nicht eingeführt werden kann.

Bei gekrümmten Prothesenschäften besteht die Problematik vor allem darin, daß einerseits genügend Raum vorhanden sein muß, um den gekrümmten Prothesenschaft trotz der im inneren Knochenraum entsprechend vorhandenen Krümmung oder Verwindung korrekt einführen zu können und andererseits im eingesetzten Zustand ein fester Sitz der Prothese — insbesondere bei zementfreier Implantation-sicherzustellen ist.

Den in den Ansprüchen 1 bis 4 angegebenen Verfahren liegt die Aufgabe zugrunde, individuelle, an die vorgefundenen oder erzeugten Knochenhohlräume optimal angepaßte Prothesen unter berücksichtigung von deren Einsetzbarkeit bei Krümmungen der betreffenden Hohlräume zu ermöglichen.

Diese Problematik stellt sich immer dann, wenn die zur Verfügung stehende Knochenöffnung kleiner ist als der durch Projektion zu erzeugende Schattenriß der notwendigen Endoprothese — die in diesem Fall nicht mittels einer linearen rotationsfreien Bewegung einführbar ist.

Die Erfindung beruht auf der Erkenntnis, daß bei der Verwendung von Prothesen eine Mindesttragfähigkeit berücksichtigt werden muß, welche entsprechend eine Mindestbaugröße der Prothese erfordert, wobei diese geforderte Mindesttragfähigkeit eine unterschiedliche Formgestaltung annehmen kann und besonderen Anforderungen noch durch die Auswahl des Werkstoffes innerhalb der für Implantationszwecke zur Verfügung stehenden körperverträglichen Werkstoffe Rechnung getragen werden kann.

Durch die Erzeugung des Modells der Knochenhöhlung besteht die Möglichkeit — vorzugsweise mittels Simulation durch einen Computer — den Weg des optimalen Einführens und die beste Form des Schaftes zu bestimmen. Dabei wird durch die verdrängbare Konsistenz der Prothesenaußenkontur, welche außerhalb der für die Tragfähigkeit erforderlichen minimalen Festigkeitszone gelegen ist, am Modell ermittelt, welche Bereiche des Prothesenrohlings abzutragen sind. Der Prothesenrohling braucht damit für alle vorkommenden Fälle nur jeweils in einer Ausführung vorhanden zu sein — es sei denn es sollen auch unterschiedliche Werkstoffe zur Ver wendung kommen. Die Mindeststruktur zur Aufrechterhaltung der Tragfähigkeit der Prothese ist in den zur Verwendung kommenden Modellen ebenfalls herausmodelliert. Diese Mindeststruktur muß erhalten bleiben, so daß eine Verklemmung, die ein Einführen der Prothese innerhalb dieser Mindeststruktur verhindert, durch Abtragung von Knochenmaterial ermöglicht werden muß. Insbesondere durch Vorsehen unterschiedlicher Formen dieser Mindeststruktur kann die somit noch notwendige Abtragung von Knochenmaterial auf ein Minimum reduziert werden.

Unter "Prothesenrohling" ist hier zweckmäßigerweise eine Ausgangsform zu verstehen, welche die maximale Größe eines für den betreffenden Anwendungsfall in Betracht kommenden Typs bildet. Letztendlich könnte aber auch von einem beliebigen Rohling ausgegangen werden, wenn der notwendige Materialabtrag in Kauf genommen wird.

Die Ausführung der erfindungsgemäßen Verfahren läßt damit unterschiedliche Varianten zu:

bei einer ersten Variante ist das einzuführende Modell der Prothese zweischichtig ausgebildet, in seinen inneren und äußeren Teilen eine unterschiedliche Konsistenz. Die äußeren Bereiche sind leichter verdrängbar, so daß sie beim Einführen in das Modell der Knochenhöhlung beseitigt werden. Die Konsistenz des inneren Kernes mit der Mindeststruktur hat eine höhere Festeigkeit (kleinere Verdrängbarkeit) als das Innere des Modells der Knochenhöhlung. Bei diesem ersten Modell würde also das Volumen der äußeren bereiche der Prothese durch das Modell der Knochensubstanz und das Volumen des Modells der Knochenhöhlung durch den Kernbereich der Prothese verdrängt werden. Das Einführen der Prothese erfolgt dabei auf einem Weg der geringsten Verdrängung vom Volumen der Knochenhöhlung jeweils bezogen auf kleine (differentielle) Vorschübe der Prothese innerhalb der Höhlung.

Auf diese Weise entsteht nach Beseitigung des verdrängbaren Materials im äußeren Bereich des Modells des Prothesenschaftes eine Vorlage zum Erzeugen der realen Prothese mittels eines numerisch gesteuerten Fräsautomaten aus einem vorgegebenen Rohling.

Der beim Einführen der fertigen prothese einzuschlagende Weg und die dabei notwendige räumliche Orientierung sowie Ort und Menge des möglicherweise noch zu entfernenden Knochenmaterial werden dokumentiert und dem behandelnden Chirugen für die Operation zur Verfügung gestellt. Auf diese Weise ist ein optimaler Sitz des so gefertigten Prothesenschaftes sichergestellt. Berücksichtigt ist dabei auch, daß die günstigste Form des Prothesenschaftes — ins besondere bei Hüftgelenkprothesen — unter Ausnutzung der gegebenen anatomischen Verhältnisse bevorzugt eine Rotation beim Einführen verlangt, der mittels der erfindungsgemäßen Verfahren in besonders vorteilhafter Weise Rechnung getragen werden kann.

Wenn davon ausgegangen werden kann, daß Substanz des Knochens nicht entfernt zu werden braucht, ist lediglich ein homogenes Prothesenmodell erforderlich, (Anspruch 1) wobei ausschließlich Material dieses Schaftmodells verdrängt wird. Nach erfolgtem Einführen des Modells kann gegebenenfalls überprüft werden, ob der verbleibende "Restschaft" noch die notwendige Festigkeit aufweist.

Bei einer dritten Ausführungsvariante wird diese Überprüfung durch ein zweites Schaftmodell vorgenommen, welches in ein Modell der Knochenbildung mit einer Konsistenz mittlerer Verdrängbarkeit eingeführt wird, so daß bei dieser Ausführung des Verfahrens die beiden Schichten des Schaftmodells gemäß dem ersten Verfahren praktisch nacheinander ausgeführt werden.

Steht hierbei neben einem festen Werkstoff lediglich ein solcher einer einzigen Verdrängbarkeitsklasse zur Verfügung, so werden die beiden Einführungen nacheinander mit zwei bezüglich ihrer Form übereinstimmenden Knochenmodellen ausgeführt, von denen eines fest und das andere verdrängbar ist. Der Begriff "verdrängbar" soll im Zusammenhang mit der Erfindung stets synomym mit "beseitigbar" verstanden werden, da es für das erfindungsgemäße Verfahren nicht von Bedeutung ist, wo der abgetragene Werkstoff verbleibt. Insbesondere für die Computersimulation wird er als beseitigt angesehen. Bei Realisierungen einer Vorrichtung zur Durchführung des Verfahrens kann das "Abtragen" bevorzugt auch in der Weise erfolgen, daß das jeweils widerstandsfähigere oder härtere Modell durch eine Abtragungsvorrichtung gebildet wird, welche von dem "weicheren" Modell den "verdrängten" Teil entfernt, was bevorzugt durch einen numerisch gesteuerten Fräsautomaten oder ein ensprechendes Laser-Schneidgerät erfolgen kann, wenn der jeweilige Modell — Werkstoff entsprechend ausgewählt ist.

Bei der Ermittlung der Form einer Prothese mit ausreichender Mindestfestigkeit ist auch noch die Festigkeit des verbleibenden Knochenmaterials in betracht zu ziehen. Weiterhin muß bei der Wahl eines Prothesenmodells, welches eine notwendige Mindestfestigkeit aufweist, das jeweilige Körpergewicht des Patienten berücksichtigt werden. Diese zusätzlichen Bedingungen beeinflussen aber nicht den grundsätzlichen Weg der Anpassung eines Prothesenschafts nach dem erfindungsgemäßen Verfahren.

Die Erfindung kann in beliebiger weise ausgeführt werden, wobei die Modellerzeugung entweder konkret räumlichkörperlich mittels werkstoffverdrängender oder abtragender Werkzeuge oder aber durch Computersimulation vorgenommen werden kann. Die differentielle räumliche Verdrängung des Werkstoffes der Prothese oder Oberfläche des Knocheninnenraumes wird dabei durch eine Ermittlung der für den Vorschub erforderlichen Kraft vorgenommen, wobei durch geringfügige Veränderung der Vorschubrichtung (Rotation) der Prothese beim Einführen oder deren Richtungsänderung die Orientierung und Richtung des Vorschubs mit geringstem Kraftaufwand festgestellt wird, so daß ein Einschieben mit minimalem Werkstoffabtrag erfolgt.

Bei der Durchführung des modellmäßige Anpassens mittels elektronischer Datenverarbeitung wird von einem System mit "Computer — Aided — Design (CAD) Gebrauch gemacht, wobei eine räumliche Durchdringung der beiden Modelle erfolgt. Dabei wird der dem Restkörper zugewandte Teil des Körpers mit der höheren Verdrängbarkeit als beseitigt angenommen und eliminiert. Die Steuerung der Vorschubrichtung und die dabei einzuhaltende räumliche Orientierung des Prothesenmodells wird durch Ermittlung des jeweils beim Vorschub verdrängten Prothesenmaterials über differentielle Weglängen festgestellt. Der zurückgelegte Weg beim Einführen wird in einem entsprechenden Speicher festgehalten, wobei ein räumliches Koordinatensystem zur Orientierung dient.

Die erfindungsgemäßen Verfahren bzw. die entsprechenden Vorrichtungen zu deren Durchführung ermöglichen auf mannigfaltige Weise eine Optimierung des Prothesensitzes durch genaue Anpassung von Prothesenform und Knochenabtrag.

Das gemeinsame Grundprinzip bei den zweistufigen Verfahren besteht darin, daß zum einen der notwendige Materialabtrag der Prothese ermittelt wird, zum anderen aber die Einführbarkeit einer Prothesenform minimaler Mindestfestigkeit durch zusätzliche Knochenabtragung gewährleistet wird. Der bei der Einführung der Prothese einzuschlagende Weg — ein — schließlich Richtungsänderungen und Drehbewegungen — wird bei einer Form der erfindungsgemäßen Verfahren bei der Ermittlung des wegs mit minimalem Materialabtrag bei der Prothese ermittelt. Ausgegangen wird dabei von einem Prothesenrohling, dessen Form allgemeinsten Anwendungen angepaßt ist.

Sollte sich jedoch herausstellen, daß das Prothesenmodell mit minimaler Mindestfestigkeit beim Einführen die Abtragung von Knochenmaterial erforderlich macht, so wird bei einer anderen bevorzugten Ausführung des erfindungsgemäßen Verfahrens der Einführungsweg der Prothese derart festgelegt, daß ein minimaler Abtrag von Knochenmaterial erforderlich ist, sobald festgestellt ist, daß eine derartige Knochenmaterialabtragung erforderlich ist. Die Festlegung des erforderlichen Materialabtrags wird dann auf dem letztgenannten Weg wiederholt.

Bei einer anderen Ausführungsvariante der Erfindung wird die Menge des abzutragenden Knochenmaterials noch dadurch verringert, daß mit verschiedenen Prothesenmodellen minimaler Mindestfestigkeit, welche eine unterschiedliche Formgebung aufweisen oder aber denen werkstoffe unterschiedlicher Festigkeit zugrunde liegen, nacheinander auf dem Weg minimalen Knochenabtrags eingeführt werden, wobei abschließend diejenige Form minimaler Mindestfestigkeit gewählt wird, auf dem insgesamt der Abtrag geringsten Knochenvolumens festgestellt wurde.

Die vorstehend dargestellte Wegermittlung kann nacheinander oder — bei Verwendung von Mehrschichtmodellen — gleichzeitig erfolgen. Lediglich bei den Verfahrensstufen zur Festlegung eines Weges oder einer Prothesenform mit minimaler Mindestfestigkeit unter Berücksichtung des minimalen Knochenabtrags ist ein rekursives Verfahren insoweit erforderlich, als nacheinander verschiedene Formen minimaler Mindestfestigkeit erprobt werden sollen oder aber der Materialabtrag beim Prothesenrohling durch den Weg minimaler Materialabtragung von Knochenmaterial beeinflußt wird.

Einen anderen Aspekt der Erfindung bildet die Umwandlung der die Knochenöffnung betreffenden Daten von der Erfassung am Patienten in solche Größen, welche die Erzeugung eines Modells ermöglichen. Grundsätzlich erfolgt die Ermittlung dieser Daten aufgrund von Röntgenbildern, welche jedoch jeweils nur eine zweidimensionale Darstellung erlauben. Mittels eines Rechners, der nach dem Prinzip des "Computer-Aided-Design" (CAD) arbeitet, ist es jedoch möglich, aus mehreren zweidimensionalen Darstellungen das zugehörige dreidimensionale Modell festzulegen. Entsprechend wird auch bei zweidimensionalen Darstellungen verfahren, welche mittels eines Computertomografen erzeugt werden. Insoweit wird hinsichtlich der Ausführung auf die DE-32 13 434-Cl verwiesen.

Besonders vorteilhaft ist jedoch die direkte Umwandlung von dreidimensionalen Abbildungen in das Modell des Knochenhohlraumes. Dabei sind bevorzugt zwei Wege gangbar: Der eine besteht darin, daß eine direkte Digitalisierung einer mittels Computertomogramm erzeugten dreidimensionalen Abbildung erzeugt wird. Eine andere besteht darin, daß während der Operation der Knocheninnenraum, der für die Einführung der Prothese vorgesehen ist, abgetastet wird und durch "On — line" — Verknüpfung mit dem zugehörigen Rechner und der nachgeschalteten Werkzeugmaschine sofort eine passende Prothese erzeugt wird, welche aus der sterilen Herstellung direkt in den Operationssaal gelangt.

Vorteilhafte Weiterbildungen der Erfindung sind in den Unteransprüchen gekennzeichnet bzw. werden bei der nachstehenden Darstellung zusammen mit einer bevorzugten Ausführung der Erfindung näher beschrieben.

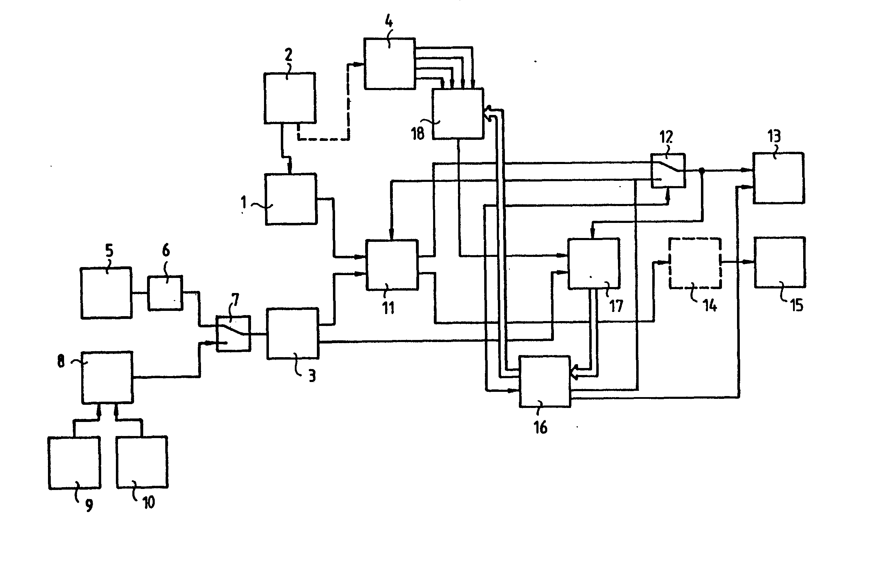

Die einzige Figur zeigt ein grundsätzliches blockschaltbild an dem die verschiedenen dargestellten Ausführungsvarianten der Erfindung näher beschrieben werden sollen.

In der Figur ist eine Baugruppe 1 zur Erzeugung eines Modells der Außenoberfläche des Prothesenrohlings wiedergegeben, wobei diese baugruppe bei räumlich — körperlicher Realisierung ein oder mehrere vorgefertigte Modelle des Prothesenrohlings enthält, der im Maßstab 1:1 oder einer entsprechenden Verkleinerung die Außenkonturen der Ausgangsformen repräsentiert. Bei räumlich — körperlicher Realisierung bestehen diese Prothesenmodelle aus einem verdrängbaren, d.h. plastisch verformbaren, Werkstoff. Bei der Ausführung mittels Simulation durch eine Datenverarbeitungsanlage sind Speicher vorgesehen, welche die Daten der Oberflächenkontur enthalten oder aber bestimmten räumlichen Punkten zugeordnet sind und wenn sie eine Information enthalten, auf die Tatsache hinweisen, daß der zugeordnete Raumpunkt von dem Prothesenrohling ausgefüllt ist.

Über einen Eingabekanal ist die Form dieses Rohlings veränderbar und kann den jeweiligen Bedürfnissen angepaßt werden. Grundsätzlich ist jedoch im wesentlichen eine einzige Ausgangssform für alle vorkommenden Anwendungsfälle vorgesehen.

Die Eingabe kann bei simulierter Verarbeitung durch Einlesen von entsprechenden Aufzeichnungsträgern gegebenenfalls aber auch durch Einlegen des jeweiligen Rohlings in eine Abtastvorrichtung, welche nach Art eines sogenannten Grafik — Tabletts eine Datenrepräsentation des Prothesenrohlings liefert.

Von der Eingabe Baugruppe 2 gelangen die Daten des Prothesenrohlings auch in eine Baugruppe 4, in der die Festigkeitsdaten des betreffenden Prothesenrohlings gespeichert werden. Insbesondere werden ein oder mehrere dreidimensionale Modelle gespeichert, die einzeln abrufbar sind und jeweils eine Formgebung repräsentieren, die an eine Prothese zu stellenden Mindestfestigkeitsanforderungen garantieren. Dabei überlagern sich die Modelle, welche in den Baugruppen 1 und 4 festgehalten werden, schalenartig, wobei das Modell in der baugruppe 1 die äußere Schale bildet.

Die in die baugruppe 3 des Modells des Knochenhohlraumes gelangenden Daten werden alternativ über verschiedene Maßnahmen zur Digitalisierung von Röntgenbildern gewonnen. Eine weitere Möglichkeit der Eingabe besteht im Abtasten von zweidimensionalen Röntgenbildern mittels eines schematisch als block dargestellten Grafiktabletts 5 Mit diesem Grafiktablett werden die punkte der Konturen des Innenhohlraumes des Knochens, in den die Prothese einzuführen ist, punktweise abgetastet und in eine nachfolgende Baugruppe 6 eingespeichert, die nach der Methode des Computer-Aided-Designs aus mehreren aus unterschiedlichen Richtungen aufgenommenen Röntgenbildern eine dreidimensionale Darstellung des Knocheninnenraumes erzeugt.

In entsprechender Weise wird bei zweidimensionalen bildern verfahren, welche von Computertomografen ausgegeben werden.

Diese Art der Ansteuerung wird gewählt, wenn sich ein Schalter 7, der für die erforderliche Datenverknüpfung sorgt, in seiner oberen Stellung befindet.

Eine andere vorteilhafte betriebsvariante des dargestellten Ausführungsbeispiels ergibt sich in der unteren Position des Umschalters 7. Hierbei werden die Daten von vornherein mit dreidimensionaler Information erfaßt, wobei lediglich noch eine Digitalisierung erforderlich ist. Diese Umwandlung der analogen räumlichen Daten, welche auf ein frei vorgebbares Koordinatensystem bezogen sind, erfolgt mit einer Digitalisier — Baugruppe 8.

Die direkt dreidimensional einzugebenden Daten werden entweder von einem Computer — Tomografen erzeugt oder über einen räumlichen Abtaster 10, der in seiner Funktionsweise dem Graphiktablett 5 entspricht, diesem gegenüber aber die Möglichkeit bietet, auch Daten einer dritten räumlichen Koordinate zu ermitteln und auszugeben.

Sowohl der Computer — Tomograf 9 als auch der räumliche Abtaster 10 können direkt in der Operationsphase eingesetzt werden und dazu dienen, die während der Operationsphase entweder röntgenografisch oder durch direkte Abtastung ermittelten Daten über die beschaffenheit des die Prothese aufnehmenden Knochenhohlraumes zur Erzeugung eines entsprechenden Modells zu verwenden.

Das Modell des Knochenhohlraumes 3, welches in der baugruppe 3 festgehalten oder durch diese erzeugt wird, und das Modell des Prothesenrohlings, welches entsprechend für die baugruppe 1 vorhanden ist, wird — wie erläutert — entweder ausschließlich datenmäßig festgehalten oder aber als räumliches Modell aus einem plastisch verformbaren Werkstoff erzeugt. Derartige plastisch verformbare Werkstoffe sind mit unterschiedlichen Plastizitätseigenschaften bekannt. Als "Verdrängbarkeit" wird hier der Widerstand angenommen, welcher ein bestimmtes Werkstoffvolumen, das mit einem anderem Werkstoff an einem vorzugsweise ebenen Flächenbereich verbunden ist, einer auf dieses Volumen wirkenden parallel zu dieser Fläche gerichteten Scherkraft entgegensetzt.

Die Modelle gemäß den Baugruppen 1 und 3 werden aus den verformbaren Werkstoffen durch numerisch gesteuerte Werkzeugmaschinen erzeugt, wie sie zur Herstellung von dreidimensionalen Körpern auch auf anderen technischen Gebieten gebräuchlich sind.

Eine erste Durchdringung erfolgt in einer baugruppe 11, wobei mittels einer geeigneten Vorrichtung das Modell des Prothesenrohlings der Baugruppe 1 in das Modell des Knochenhohlraumes der baugruppe 3 eingeführt wird. Das Modell des Knochenhohlraumes ist dabei in bezug auf die äußeren Bereiche des Prothesenmodells als fest anzusehen, während das Prothesenmodell "verdrängbar" ist. Unter Messung des für das Voranschieben jeweils erforderlichen Kraftaufwandes und dessen Minimierung im differentiellen bereich unter Wechsel von Prothesenrichtung und Orientierung in bezug auf das Modell des Knochenhohlraumes wird derjenige Weg ermittelt, der ein Einführen mit geringstem Widerstand, d.h. geringstem Materialabtrag des Prothesenrohlings ermöglicht.

Beim Eindringen des Modells des Prothesenrohlings in den Knochenhohlraum wird das Material des Prothesenrohlings in der geschriebenen Weise verdrängt, da diesen gegenüber das Material des Knochenhohlraums relativ als "fest" anzusprechen ist. Der beim Einführen ermittelte Weg minimaler Volumenverdrängung für den Prothesenrohling wird festgehalten und über ein Schaltelement 12 in eine Baugruppe 13 zur Steuerung des späteren Einführens der Prothese weitergegeben.

Es ist nun aber erforderlich, zu überpüfen, ob eine nach dem derart in seiner Volumenstruktur verringerten Prothesenmodell gefertigte reale Prothese noch den zu stellenden Festigkeitsanforderungen genügt. Zu diesem Zweck sind in der Baugruppe 4 Prothesenmodelle festgehalten, welche bei minimalen Abmessungen bei Herstellung aus Titan oder gegebenenfalls anderen körperverträglichen Werkstoffen die notwendige Festigkeit aufweisen.

Der Einführungsvorgang wird beendet, wenn die Position eines mit dem Prothesenrohling im bereich seines Schaftes verbundenen bezugspunktes eine vorgegebene Position relativ zum Knochen erreichte hat. Die verbleibende Oberflächengestaltung des Prothesenrohlings wird je nach Ausführungsform als körperliches Modell der in der Datenrepräsentation in eine Steuerbaugruppe 14 überführt, wo sie als Muster zur Ansteuerung einer Fräsmaschine 15 zum Erzeugen der realen Prothese dient. Im Falle des körperlichen Modelles handelt es sich dabei um eine herkömmliche Kopierfräsmaschine, in die das Modell der Prothese eingegeben wird, nachdem es vorher in üblicher Weise einem Aushärtungsvorgang unterworfen wurde.

Bei einer in der Zeichnung nicht dargestellten Ausführungsvariante einer Vorrichtung zur Durchführung des erfindungsgemäßen Verfahrens wird zur Festigkeitsermittlung zwischen dem angepaßten Modell des Prothesenrohlings und den Festigkeitsmodellen in der Baugruppe 4 ein Vergleich durchgeführt. Mindestens eines der vorhandenen Festigkeitmodelle muß sich vollkommen in das erhaltene Modell einbeschreiben lassen, um sicherzustellen, daß den Festigkeitsanforderungen genüge getan ist. Verläuft die entsprechende Abfrage positiv — wobei beim Vorhandensein von mehreren Festigkeitsmodellen sich lediglich eines von diesen in das Fertigungsmodell einbeschreiben lassen muß — so kann der Herstellungprozeß beginnen und es sind keine weiteren Maßnahmen erforderlich. Im anderen Fall verläuft das Verfahren so, wie es nachfolgend dargestellt ist, wenn keine Zwischenkontrolle vorgenommen wird. Nach Abschluß des Einführens des Rohlingmodells durch die Baugruppe 11 wird an einen Steuerbaustein 16 ein Ausgangssignal abgegeben, welches ein Einführen eines in der baugruppe 4 enthaltenden Festigkeitsmodells in das Modell des Knochenhohlraums in entsprechender Weise veranlaßt. In diesem Fall ist jedoch der Knochenhohlraum verdrängbarer als das Festigkeitsmodell. Letzteres ist in bezug auf den Werkstoff des Knochens fest. Die Steuerung der Einführungsrichtung und das Festhalten des beim Einführen zurückgelegten Weges erfolgt in diesem Fall in einer baugruppe 17, durch die der entsprechende Einführungsvorgang gesteuert wird. Der Prothesenweg (einschließlich Rotationen und Richtungsänderungen) wird dabei so festgelegt, daß das beim Voranschieben zu verdrängende Material des Modells des Knocheninnenraums zu einem Minimum wird.

Ist beim Einführen des Festigkeitsmodells kein Abtrag von Volumen des Knochenhohlraums erforderlich, so wird automatisch mittels der Steuerbaugruppe 14 die Herstellung der realen Prothese mittels der Fräsmaschine 15 aufgrund des mittels der Baugruppe 11 erzeugten Produktionsmodells eingeleitet.

Wurde jedoch Knochenmaterial entfernt, so wird von der Baugruppe 17 ein Ausgangssignal an den Steuerteil 16 ausgegeben, welcher über eine Auswableinheit 18 aus der Baugruppe 4 ein anders gestaltetes Festigkeitsmodell auswählt, da durch Veränderung von Krümmungen und dergleichen durchaus verschiedene Prothesenmodelle mit minimalen Abmessungen erzeugt werden können, welche den geforderten Festigkeitsanforderungen genügen. Mit diesem Modell wird der Einführungsvorgang mit minimaler differentieller Volumenverdrängung des Modells des Knochenhohlraums und der dabei zurückgelegte Weg eingespeichert. Nachdem dasjenige Modell gefunden ist, bei dem ein minimaler Abtrag von Knochenmaterial notwendig ist, wird durch den Ausgang des Steuerbaustein 16 der Umschalter 12 betätigt und somit die ermittelten Wegdaten in die Baugruppe 13 zur Steuerung beim Einführen der Prothese übertragen.

Die selben Daten werden aber auch zur Baugruppe 11 übertragen, woraufhin der Vorgang des Einführens des Prothesenrohlings mit demjenigen Modell des Knochenhohlraumes wiederholt wird, bei dem durch das Einführen des Festigkeitsmodells am wenigsten Volumen verdrängt wurde. Dieses Knochenmodell muß bei räumlich/körperlicher Realisierung wiederum fest sein gegenüber dem Modell des Prothesenrohlings, so daß nach dessen Einführen auf dem Weg, wie er für das Festigkeitsmodell bei minimaler Entfernung von Knochensubstanz gefunden wurde, durch Volumenverdrängung das Modell des Rohlings wiederum zu einem Fertigungsmodell für eine prothese umgestaltet wird, welche sich unter Berücksichtigung der vorzunehmenden Wegänderungen und Rotationsbewegungen in den durch Abtrag von Knochenmaterial veränderten Hohlraum im Knochen des Patienten einbringen läßt.

Der Ort des abzutragenden Knochenmaterials wird dem behandelnden Chirurgen über das Steuergerät zur Führung beim Einführung der Prothese 13 auf einem Sichtgerät oder dergleichen angezeigt, so daß er die entsprechenden Korrekturen des Knocheninnenraums vornehmen kann. Bei einer günstigen weiterbildung der Erfindung kann eine Kontrolle und ein Vergleich zwiscen vorgegebenem Knocheninnenraum und den Soll-Daten mittels der Baugruppe 9 oder 10 erfolgen.

Es ist ersichtlich, daß bei anderen — nicht dargestellten — Varianten des erfindungsgemäßen Verfahrens mit einem zweischichtigen Modell des Prothesenrohlings, dessen fester Kern dem Festigkeitsmodell entsoricht und dessen äußerer Schalenbereich leicht verdrängbar ist, die wege mit minimaler Entfernung von Knochensubstanz und möglichst geringer Abarbeitung der Oberfläche des Prothesenrohlings miteinander kombinierbar sind und in einem Arbeitsgang durchgeführt werden können, wenn das Modell des Innenraums des Knochens eine mittlere Verdrängbarkeit aufweist. Insbesondere bei der Simulation durch ein Computermodell läßt sich auf diese weise eine hohe Verarbeitungsgeschwindigkeit erreichen, da die Zahl von umfangreichen Operationen zur Datenverschiebung erspart ist, wenn die örtlichen Modelldaten bei einem Arbeitsvorgang mehrfach benutzt können. Bei der Datenverarbeitung und insbesondere bei den Verfahren des Computer-Aided-Design werden sich die räumlichen Modelle beim Voranbewegen des Prothesenmodells zum Einführung in den Knochenhohlraum nicht verdrängen, sondern durchdringen. In diesem Fall wird dasjenige Volumen, welches im Vorstehenden als im Vergleich zu dem anderen Körper "verdrängbar" bezeichnet ist, als beseitigt gekennzeichnet, so daß insoweit im wesentlichen dieselben Überlegungen maßgeblich sind.

Die Orte der gegenseitigen Durchdringung auf dem Wege des Einführens des Prothesenmodells in das Knochenmodell werden in entsprechenden Speicherbereichen des Simulationsrechners durch geeignete binäre Signalzustände gekennzeichnet. Damit sind bevorzugt diejenigen Bereiche dem Chrirurgen mittels Display oder Plotter graphisch darstellbar, bei denen ein Knochenabtrag erforderlich ist. Bei minimalem rechnerischen Aufwand lassen sich auf dem weg des Einschiebens die räumlichen Bereiche der Durchdringung kumulieren. In dem betreffenden Speicher wird einfach jedem in betracht kommenden Raumpunkt ein Speicherplatz zugeordnet und der Signalzustand des Speicherplatzes in einem bit geändert, wenn der jeweilige Raumpunkt von einer Durchdringung von Prothesen und Knochenmodell betroffen wurde. Durch Vergleich der graphischen Darstellungen der so gefundenen Durchdringungsbereiche bei der Auswahl unterschiedlicher Modelle mit vorgegebener Mindestfestigkeit kann der Chirurg aus der Anschauung entscheiden, welche von verschiedenen Prothesenformen nach seinem Empfinden — bei ohnehin notwendigem Knochenabtrag — individuell die besten Voraussetzungen bietet. Trotz automatisierter Formanpassung läßt sich somit bei verschiedenen möglichen gleichwertigen Lösungen die Erfahrung des Arztes mit verschiedenen Formen und deren Eigenschaften — bei rechnerisch nahezu identischen Eigenschaften der ermittelten prothesenformen — mit in betracht ziehen.

Bei einer anderen — in der Zeichnung nicht dargestellten — Variante der Erfindung werden die von einem Computertomografen ermittelten digitalisierten Daten mit entsprechenden Schaltungsmitteln über das Fernsprechnetz übertragen und nach dem erfindungsgemäßen Modellabgleich einem Fräsautomaten zugeleitet. Die Übertragung erfolgt bevorzugt über ein Datennetz oder einen für die Datenübertragung vorbereiteten Nachrichtenkanal, wie er beispielsweise durch das System Bildschirmtext gegeben ist.

**Patentansprüche**

1. Verfahren zur Herstellung einer Endoprothese mit in- dividueller Anpassung, insbesondere mit einem gekrümmten Prothesenschaft, gekennzeichnet durch
die Schritte:
a. dreidimensionale Ausmessung der Knochenhöhlung, ohne daß dieser Ausmessung ein chirurgischer Eingriff vorausgeht,
b. Erzeugung eines festen Modells der Knochenhöhlung,
c. Erzeugung eines durch das Modell der Knochenhöhlung verdrängbaren Modells der Prothese, deren äußere Abmessungen derjenigen des Prothesenrohlings entsprechen,
d. Eindringen des Prothesenmodells in das Modell der Knochenhöhlung auf einem Weg kleinster Volumenverdrängung, gegebenenfalls unter Einschluß von Rotationsbewegungen und Richtungsänderungen, durch Minimierung des beim Vorschub, pro Wegeinheit verdrängten Volumens und insbesondere Aufzeichnung des so zurückgelegten Wegs einschließlich der räumlichen Ausrich- tung der Prothese auf diesem Weg,
d1. Überprüfung der Festigkeit des verbleibenden Prothesenmodells, bezogen auf den realen Prothesenwerkstoff, sowie
e. Steuerung des Entfernens des beim Prothesenmodell verdrängten Volumens auch beim realen Prothesenrohling durch spanabhebende Verformung mittels einer numerisch gesteuerten Werkzeugmaschine.

2. Verfahren zur Herstellung einer Endoprothese mit individueller Anpassung, insbesondere mit einem gekrümmten Prothesenschaft, gekennzeichnet durch
die Schritte:
a. dreidimensionale Ausmessung der Knochenhöhlung, ohne daß dieser Ausmessung ein chirurgischer Eingriff vorausgeht,
b. Erzeugung eines Modells der Knochenhöhlung, dessen Konsistenz eine mittlere Verdrängbarkeit aufweist,
c. Erzeugung eines Modells der Prothese, bestehend aus einem festen Kernbereich, der in seinen Abmessungen den Mindestabmessungen der aus dem realen körperverträglichen Werkstoff bestehenden Prothese entspricht, wobei die Mindestabmessungen unter Berücksichtigung der notwendigen Tragfähigkeit der Prothese festgelegt sind, und einem äußeren Bereich mit einer Konsistenz hoher Verdrängbarkeit, dessen äußere Abmessungen dem prothesenrohling entsprechen,
d. Eindringen des Prothesenmodells in das Modell der Knochenhöhlung auf einem Weg kleinster Volumenverdrängung des Modells der Knochenhöhlung, gegebenenfalls unter Einschluß von Rotationsbewegungen und Richtungsänderungen, durch Minimierung des beim Vorschub pro Wegeinheit verdrängten Volumens und Auf- zeichnung des so zurückgelegten Wegs einschließlich der räumlichen Ausrichtung der prothese auf diesem Weg,
e. Steuerung des Entfernens des beim Prothesenmodell verdrängten Volumens auch beim realen Prothesenrohling durch spanabhebende Verformung mittels einer numerisch gesteuerten Werkzeugmaschine.

3. Verfahren zur Herstellung einer Endoprothese mit individueller Anpassung, insbesondere mit einem gekrümmten Prothesenschaft,
gekennzeichnet durch die Schritte:
a. dreidimensionale Ausmessung der Knochenhöhlung, ohne daß dieser Ausmessung ein chirurgischer Eingriff vorausgeht,
b. Erzeugung eines Modells der Knochenhöhlung mit einer Konsistenz mittlerer Verdrängbarkeit,
c. Erzeugung eines ersten festen Modells der Prothese, das in seinen Abmessungen den Mindestabmessungen des aus dem realen körperverträglichen Werkstoff bestehenden Prothesenschaftes entspricht, wobei die Mindestabmessungen unter Berücksichtigung der notwendigen Tragfähigkeit der Prothese festgelegt sind, und
d. Eindringen des ersten Prothesenmodells in das Modell der Knochenhöhlung auf einem Weg kleinster Volumenverdrängung, gegebenenfalls unter Einschluß von Rotationsbewegungen und Richtungsänderungen, durch Minimierung des beim Vorschub pro Wegeinheit verdrängten Volumens und Aufzeichnung des so zurückgelegten Wegs, einschließlich der räumlichen Ausrichtung der Prothese auf diesem Weg,

c'. Erzeugung eines zweiten Prothesenmodells mit einer Konsistenz hoher Verdrängbarkeit, dessen äußere Abmessungen denen des Prothesenrohlings entsprechen,

d'. Eindringen des zweiten Prothesenmodells in das Modell der Knochenhöhlung auf einem Weg mit einer jeweiligen Ausrichtung entsprechend dem Weg und der jeweiligen Ausrichtung des ersten Modells, sowie

e. Steuerung des Entfernens des beim zweiten Prothesenmodell verdrängten Volumens auch beim realen prothesenrohling durch spanabhebende Verformung mittels einer numerisch gesteuerten Werkzeugmaschine.

4. Verfahren zur Herstellung einer Endoprothese mit individueller Anpassung, insbesondere mit einem gekrümmten Prothesenschaft, gekennzeichnet durch
die Schritte:

a. dreidimensionale Ausmessung der Knochenhöhlung, ohne daß dieser Ausmessung ein chirurgischer Eingriff vorausgeht.

b. Erzeugung eines Modells der Knochenhöhlung mit verdrängbarer Konsistenz,

c. Erzeugung eines ersten festen Modells der Prothese, das in seinen Abmessungen den Mindestabmessungen der aus dem realen körperverträglichen Werkstoff bestehenden Prothese entspricht, wobei die Mindestabmessungen unter Berücksichtigung der notwendigen Tragfähigkeit der Prothese festgelegt sind, und

d. Eindringen des ersten prothesenmodells in das Modell der Knochenhöhlung auf einem Weg kleinster Volumenverdrängung, gegebenenfalls unter Einschluß von Rotationsbewegungen und Richtungsänderungen, durch Minimierung des beim Vorschub pro Wegeinheit verdrängten Volumens und Aufzeichnung des so zurückgelegten Wegs, einschließlich der räumlichen Ausrichtung der Prothese auf diesem Weg,

b'. Erzeugung eines zweiten festen Modells der Knochenhöhlung mit der Oberflächenformung des ersten Modells nach Eindringen des ersten Prothesenmodells bzw. Härten des ersten Modells,

c'. Erzeugung eines zweiten Prothesenmodells mit verdrängbarer Konsistenz, dessen äußere Abmessungen denen des Prothesenrohlings entsprechen,

d'. Eindringen des zweiten Prothesenmodells in das zweite Modell der Knochenhöhlung auf einem Weg und einer jeweiligen Ausrichtung entsprechend dem Weg und der jeweiligen Ausrichtung des ersten Modells, sowie

e. Steuerung des Entfernens des beim zweiten Prothesenmodell verdrängten Volumens auch beim realen Prothesenrohling durch spanabhebende Verformung mittels einer numerisch gesteuerten Werkzeugmaschine.

5. Verfahren nach einem der vorangehenden Ansprüche, dadurch gekennzeichnet, daß die Verfahrensschritte: Erzeugung eines Modells und Eindringen des Prothesenmodells in das Modell der Knochenhöhlung mittels Simulation auf einem Rechner vorgenommen werden, wobei statt Verdrängung eine Durchdringung in der Weise zugrundegelegt wird, daß räumliche Bereiche in denen eine derartige Verdrängung auf dem genannten Weg festgestellt wurde, als entsprechende Daten im Speicher des Simulationsrechners festgehalten werden.

6. Verfahren nach Anspruch 5, dadurch gekennzeichnet, daß die Durchdringung zwischen dem Knochenmodell und dem Prothesenmodell mit der Mindestfestigkeit einem Graphik — Display oder einem plotter zwecks Darstellung zugeführt werden.

7. Verfahren nach den Ansprüchen 1, 5 und 6, dadurch gekennzeichnet, daß statt der Überprüfung der Festigkeit des verbleibenden Prothesenmodells ein Prothesenmodell mit der notwendigen Mindestfestigkeit ausgewählt und die gegenseitige Durchdringung dargestellt wird.

8. Verfahren nach einem der vorangehenden Ansprüche, dadurch gekennzeichnet, daß mehrere Prothesenmodelle, entsprechend verschiedenen Formen von Prothesen mit der notwendigen Mindestfestigkeit, gegebenenfalls unter Berücksichtigung von verschiedenen Werkstoffen unterschiedlicher Festigkeit, nacheinander auf dem Weg kleinster Volumenverdrängung geführt werden.

9. Verfahren nach einem der vorangehenden Ansprüche, dadurch gekennzeichnet, daß die Erzeugung einer Datenrepräsentation des Knochenmodells durch Abtastung mehrerer aus unterschiedlichen Richtungen aufgenommener Röntgenbilder oder einer computertomografischen Darstellung in mehreren ebenen Ansichten mittels eines zweidimensional arbeitenden Umsetzers für Wegstrecken in digitale Signale oder direkt mittels eines dreidimensional arbeitenden Umsetzers für Wegstrecken in digitale Signale erfolgt.

10. Verfahren nach Anspruch 9, dadurch gekennzeichnet, daß Schaltvorrichtungen zur digitalen Übertragung der Datenrepräsentation des Knochenmodells über eine Fernsprechleitung vorgesehen sind.

**Revendications**

1. Procédé de réalisation d'une endoprothèse avec adaptation individuelle, en particulier avec une crosse courbe de prothèse,
caractérisé par les étapes:

a. relevé tridimensionnel de la cavité de l'os, sans qu'une intervention chirurgicale précède ce relevé,

b. fabrication d'un modèle en dur de la cavité de l'os,

c. fabrication d'un modèle de la prothèse que le modèle de la cavité de l'os puisse refouler et dont les dimensions extérieures correspondent à celles de l'ébauche de la prothèse,

d. introduction du modèle de la prothèse dans le modèle de la cavité de l'os selon un chemin donnant le plus faible refoulement volumique, éventuellement en y incluant des mouvements de rotation et des modifications de direction, par réduction au minimum du volume refoulé par unité de chemin lors de l'avance, et en particulier par enregistrement du chemin ainsi parcouru, y compris de l'orientation spatiale de la prothèse sur ce chemin,

d'. vérification de la résistance du modèle de prothèse subsistant, rapportée au matériau de la prothèse réelle, ainsi que

e. commande de l'enlèvement sur l'ébauche réelle également de la prothèse, par déformation avec enlèvement de copeaux au moyen d'une machine — outil à commande numérique, du volume refoulé sur le modèle de la prothèse.

2. Procédé de réalisation d'une endoprothèse avec adaptation individuelle, en particulier avec une crosse courbe de prothèse, caractérisé par les étapes:

a. relevé tridimensionnel de la cavité de l'os, sans qu'une intervention chirurgicale précède ce relevé,

b. fabrication d'un modèle de la cavité de l'os dont la consistance présente une aptitude moyenne à subir un refoulement,

c. fabrication d'un modèle de la prothèse constitué d'une zone de noyau en dur, dont les dimensions correspondent aux dimensions minimales de la prothèse réelle constituée d'un matériau compatible avec le corps, les dimensions minimales étant déterminées en tenant compte de la résistance nécessaire de la prothèse, et

d'une zone extérieure dont la consistance correspond à une aptitude élevée à subir un refoulement et dont les dimensions extérieures correspondent à l'ébauche de la prothèse,

d. introduction du modèle de la prothèse dans le modèle de la cavité de l'os selon un chemin donnant le plus faible refoulement volumique du modèle de la cavité de l'os, éventuellement en y incluant des mouvements de rotation et des modifications de direction, par réduction au minimum du volume refoulé par unité de chemin lors de l'avance, et par enregistrement du chemin ainsi parcouru, y compris de l'orientation spatiale de la prothèse sur ce chemin

e. commande de l'enlèvement sur l'ébauche réelle également de la prothèse, par déformation avec enlèvement de copeaux au moyen d'une machine — outil à commande numérique, du volume refoulé sur le modèle de la prothèse.

3. Procédé de réalisation d'une endoprothèse avec adaptation individuelle, en particulier avec une crosse courbe de prothèse, caractérisé par les étapes:

a. relevé tridimensionnel de la cavité de l'os, sans qu'une intervention chirurgicale précède ce relevé,

b. fabrication d'un modèle de la cavité de l'os dont la consistance présente une aptitude moyenne à subir un refoulement,

c. fabrication d'un premier modèle, en dur, de la prothèse, dont les dimensions correspondent aux dimensions minimales de la crosse de la prothèse réelle constituée d'un matériau compatible avec le corps, les dimensions minimales étant déterminées en tenant compte de la résistance nécessaire de la prothèse, et

d. introduction du premier modèle de la prothèse dans le modèle de la cavité de l'os selon un chemin donnant le plus faible refoulement volumique, éventuellement en y incluant des mouvements de rotation et des modifications de direction, par réduction au minimum du volume refoulé par unité de chemin lors de l'avance, et par enregistrement du chemin ainsi parcouru, y compris de l'orientation spatiale de la prothèse sur ce chemin

c'. fabrication d'un second modèle de la prothèse dont la consistance présente une aptitude élevée à subir un refoulement et dont les dimensions extérieures correspondent à celles de l'ébauche de la prothèse.

d'. introduction du second modèle de la prothèse dans le modèle de la cavité de l'os selon un chemin et avec une orientation à tout moment correspondant au chemin et à l'orientation à tout moment du premier modèle, ainsi que

e. commande de l'enlèvement sur l'ébauche réelle également de la prothèse, par déformation avec enlèvement de copeaux au moyen d'une machine — outil à commande numérique, du volume refoulé sur le second modèle de la prothèse.

4. Procédé de réalisation d'une endoprothèse avec adaptation individuelle, en particulier avec une crosse courbe de prothèse, caractérisé par les étapes:

a. relevé tridimensionnel de la cavité de l'os, sans qu'une intervention chirurgicale précède ce relevé,

b. fabrication d'un modèle de la cavité de l'os dont la consistance présente une aptitude à subir un refoulement,

c. fabrication d'un premier modèle, en dur, de la prothèse, dont les dimensions correspondent aux dimensions minimales de la prothèse réelle, constituée d'un matériau compatible avec le corps, les dimensions minimales étant déterminées en tenant compte de la résistance nécessaire de la prothèse et,

d. introduction du premier modèle de la prothèse dans le modèle de la cavité de l'os selon un chemin donnant le plus faible refoulement volumique, éventuellement en y incluant des mouvements de rotation et des modifications de direction, par réduction au minimum du volume refoulé par unité de chemin lors de l'avance, et par enregistrement du chemin ainsi parcouru, y compris de l'orientation spatiale de la prothèse sur ce chemin

b'. fabrication d'un second modèle, en dur, de la cavité de l'os dont la forme de surface est celle du premier modèle après pénétration du premier modèle de la prothèse et traitement du premier modèle,

c'. fabrication d'un second modèle de la prothèse dont la consistance présente une aptitude à subir un refoulement et dont les dimensions extérieures correspondent à celles de l'ébauche de la prothèse,

d'. introduction du second modèle de la prothèse dans le second modèle de la cavité de l'os selon un chemin et avec une orientation à tout moment correspondant au chemin et à l'orientation à tout moment du premier modèle, ainsi que,

e. commande de l'enlèvement sur l'ébauche réelle également de la prothèse, par déformation avec enlèvement de copeaux au moyen d'une machine — outil à commande numérique, du volume refoulé sur le second modèle de la prothèse.

5. Procédé selon l'une des revendications précédentes, caractérisé en ce que l'on procède aux étapes du procédé: production d'un modèle et introduction du modèle de la prothèse dans le modèle de la cavité de l'os par simulation sur un ordinateur, procédé dans lequel, au lieu de "refoulement" on prend comme base "interpénétration" en ce sens que les zones spatiales pour lesquelles on a déterminé un refoulement de ce type sur le chemin mentionné sont conservées, sous forme de données correspondantes, dans la mémoire de l'ordinateur de simulation.

6. Procédé selon la revendication 5, caractérisé en ce que l'interpénétration entre le modèle de l'os et le modèle de la prothèse présentant la résistance minimale est envoyée à un visuel graphique ou à un traceur aux fins de représentation.

7. Procédé selon les revendications 1, 5 et 6, caractérisé en ce qu'au lieu de la vérification de la résistance du modèle de prothèse subsistant, on choisit un modèle de prothèse présentant la résistance minimale nécessaire et on représente l'interpénétration mutuelle.

8. Procédé selon l'une des revendications précédentes, caractérisé en ce que l'on amène successivement selon le chemin donnant le plus faible refoulement volumique plusieurs modèles de prothèses, correspondant à différentes formes de prothèses présentant la résistance minimale nécessaire, éventuellement en tenant compte de différents matériaux de résistance différente.

9. Procédé selon l'une des revendications précédentes, caractérisé en ce que la production d'une représentation, sous forme de données, du modèle de l'os se fait par exploration de plusieurs images obtenues par rayons X depuis des directions différentes, ou bien par exploration d'une représentation tomographique par ordinateur selon plusieurs vues planes, au moyen d'un convertisseur, travaillant en bidimensionnel, des longueurs de chemin en signaux numériques, ou bien directement au moyen d'un convertisseur, travaillant en tridimensionnel, des longueurs de chemin en signaux numériques.

10. Procédé selon la revendication 9, caractérisé en ce que les dispositifs de commutation sont prévus pour la transmission numérique de la représentation, par des données, du modèle de l'os par l'intermédiaire d'une ligne téléphonique.

**Claims**

1. A method of manufacturing an individually adapted endoprosthesis, more particularly having a curved prosthesis shaft,

characterised by the following steps:

a. three-dimensional survey of the bone cavity, without any surgical intervention preceding this survey

b. producing a solid model of the bone cavity

c. producing a model of the prosthesis which may be compressed or displaced by the model of the bone cavity, the outer dimensions of the prosthesis model matching those of the prosthesis blank,

d. forcing the prosthesis model into the model of the bone cavity on a path having the least volume compression or displacement, if necessary including rotary movements and changes of direction, by minimising the volume compressed or displaced during forward movement, per unit of the path and particularly recording the path thus travelled including the spatial alignment of the prosthesis on this path,

d1. testing the strength of the remaining prosthesis model, in relation to the real prosthesis material, and

e. controlling the removal of the volume compressed or displaced in the prosthesis model, in the real prosthesis blank too, by changing the shape by cutting with the aid of a numerically controlled machine tool.

2. A method of manufacturing an individually adapted endoprosthesis, more particularly having a curved prosthesis shaft,

characterised by the following steps:

a. three-dimensional survey of the bone cavity, without any surgical intervention preceding the survey,

b. producing a model of the bone cavity, the consistency of which has an average compressibility or displaceability,

c. producing a model of the prosthesis, comprising a solid core region, which corresponds in its dimensions to the minimum dimensions of the prosthesis which comprises the real material which is compatible with the body, in which the minimum dimensions are fixed taking account of the necessary load bearing capacity of the prosthesis, and comprising an outer region with a consistency of high compressibility or displaceability, the outer dimensions of which correspond to the prosthesis blank,

d. forcing the prosthesis model into the model of the bone cavity on a path of least volume compression or displacement of the model of the bone cavity, if necessary including rotary movements and changes of direction, by minimising the volume compressed or displaced during forward movement per unit of path and recording the path thus travelled including the spatial alignment of the prosthesis on this path,

e. controlling the removal of the volume compressed or displaced in the prosthesis model, in the real prosthesis blank too by changing the shape by cutting with the aid of a numerically controlled machine tool.

3. A method of manufacturing an individually adapted endoprosthesis, more particularly having a curved prosthesis shaft,

characterised by the following steps:

a. three-dimensional survey of the bone cavity, without any surgical intervention preceding this survey,

b. producing a model of the bone cavity with a consistency of average compressibility,

c. producing a first solid model of the prosthesis, which model corresponds in its dimensions to the minimum dimensions of the prosthesis shaft comprising the real material which is compatible with the body, in which the minimum dimensions are fixed taking account of the necessary load bearing capacity of the prosthesis and

d. forcing the first prosthesis model into the model of the bone cavity on a path of least volume compression or displacement, if necessary including rotary movements and direction changes, by minimising the volume compressed or displaced during forward movement per unit of path and recording the path thus travelled, including the spatial alignment of the prosthesis on this path,

c'. producing a second prosthesis model with a consistency of high compressibility or displaceability, its outer dimensions corresponding to those of the prosthesis blank,

d'. forcing the second prosthesis model into the model of the bone cavity on a path having a respective alignment corresponding to the path and the respective alignment of the first model, and

e. controlling the removal of the volume compressed or displaced in the second prosthesis model, in the real prosthesis blank too by changing the shape by cutting with the aid of a numerically controlled machine tool.

4. A method of manufacturing an individually adapted endoprosthesis, more particularly having a curved prosthesis shaft,

characterised by the following steps:

a. three-dimensional survey of the bone cavity, without any surgical intervention preceding this survey,

b. producing a model of the bone cavity with a compressible or displaceable consistency,

c. producing a first solid model of the prosthesis, which model corresponds in its dimensions to the minimum dimensions of the prosthesis comprising the real material which is compatible with the body, in which the minimum dimensions are fixed taking account of the necessary load bearing capacity of the prosthesis, and

d. forcing the first prosthesis model into the model of the bone cavity on a path of least volume compression or displacement, if necessary including rotary movements and direction changes, by minimising the volume compressed or displaced during forward movement per unit of path and recording the path thus travelled, including the spatial alignment of the prosthesis on this path,

b'. producing a second solid model of the bone cavity with the shaping of the surface of the first model after forcing in of the first prosthesis model or hardening of the first model,

c'. producing a second prosthesis model with compressible or displaceable consistency, its outer dimensions corresponding to those of the prosthesis blank,

d'. forcing in the second prosthesis model into the second model of the bone cavity on a path and respective alignment corresponding to the path and the respective alignment of the first model, and

e. controlling the removal of the volume compressed or displaced in the second prosthesis model, in the real prosthesis blank too, by changing the shape by cutting with the aid of a numerically controlled machine tool.

5. A method according to any one of the preceding claims, characterised in that the process steps: producing a model and forcing the prosthesis model into the model of the bone cavity are implemented with the aid of simulation on a computer, based on "penetration" instead of "compression or displacement" such that spatial regions in which such compression or displacement was established on the said path are stored in the memory of the simulation computer as appropriate data.

6. A method according to claim 5, characterised in that the penetration between the bone model and the prosthesis model having the minimum strength is passed to a graphic display or a plotter for the purpose of display.

7. A method according to claims 1, 5 and 6, characterised in that, instead of checking the strength of the remaining prosthesis model a prosthesis model is selected having the necessary minimum strength and the mutual penetration is illustrated.

8. A method according to any one of the preceding claims, characterised in that several prosthesis models, corresponding to different shapes of prosthesis with the necessary minimum strength, if necessary taking account of different materials of different strength, are guided in succession on the path of least volume compression or displacement.

9. A method according to any one of the preceding claims, characterised in that a data representation of the bone model is produced by scanning several x-rays taken from different directions or scanning a computer tomographic representation in, several planar views with the aid of a two-dimensionally operating converter for converting path distances into digital signals or is produced directly with the aid of a three-dimensionally operating converter for converting path distances into digital signals.

10. A method according to claim 9, characterised in that switching devices are provided for digital transmission of the data representation of the bone model via a telephone line.